# EUROPEAN PATENT APPLICATION

(11) **EP 4 487 919 A1**
(43) Date of publication of application: **08.01.2025**
(21) Application number: 23763566.9
(22) Date of filing: 03.03.2023
(51) Int. Cl.: A61Q 11/00, A61K 8/02, A61K 8/34

(54) **ORAL CAVITY AEROSOL AGENT**

(30) Priority: 04.03.2022 WO PCT/JP2022/009418
(71) Applicant: Kao Corporation, Tokyo 103-8210 (JP)
(72) Inventor: NISHIWAKI, Keisuke, Tokyo 131-8501 (JP)
(74) Representative: Hoffmann Eitle
(86) International application number: PCT/JP2023/007982
(87) International publication number: WO 2023/167309

(57) **Abstract**

The present invention relates to an oral cavity aerosol agent which can well protect gums by exhibiting high foaming performances. Specifically, the present invention provides an oral cavity aerosol agent comprising a stock solution (X) and a propellant (Y), the stock solution (X) comprising the following components (a), (b), and (c): (a) 0.6 mass% or more and 10 mass% or less of a monovalent alcohol having 12 or more and 22 and less carbon atoms, (b) a surfactant, and (c) water, wherein a mass ratio of a content of the component (b) to a content of the component (a), ((b)/(a)), in the stock solution (X) is 0.08 or more and 10 or less, and a content of ethanol is 8 mass% or less.

## Description

### Field of the Invention

The present invention relates to an oral cavity aerosol agent.

### Background of the Invention

Compositions for the oral cavity exist in various dosage forms, such as pastes and liquids, and products manufactured by making the best of characteristics of each form are available for practical use. Oral cavity aerosol agents are under development in which a composition for the oral cavity is filled into an aerosol container together with a propellant, such as carbon dioxide (carbonic acid gas), and squirted at the time of use, because they can take advantage of the blood flow-promoting effect and the like brought about by carbon dioxide.

For example, Patent Literature 1 discloses a foam agent for preventing and improving gingival retraction which contains a stock solution containing a medicinal ingredient, such as tocopherol acetate or glycyrrhetinic acid, and a propellant containing carbon dioxide. Here, gingival retraction is intended to be prevented or improved by applying the medicinal ingredient together with carbon dioxide to the gingiva. Patent Literature 2 discloses an oral cavity aerosol agent which contains a stock solution and a propellant contains carbon dioxide, wherein the stock solution contains a liquid dihydric alcohol, a medicinal ingredient, a nonionic surfactant, and water, in predetermined amounts and at predetermined mass ratios. Here, attempts have been made to enjoy the benefits of the medical ingredient, etc. while maintaining the health of the gingiva through the blood flow-promoting effect of carbon dioxide.

(Patent Literature 1) JP-A-2017-95382
(Patent Literature 2) JP-A-2018-104320

### Summary of the Invention

The present invention provides an oral cavity aerosol agent comprising a stock solution (X) and a propellant (Y), the stock solution (X) comprising the following components (a), (b), and (c):
(a) 0.6 mass% or more and 10 mass% or less of a monovalent alcohol having 12 or more and 22 and less carbon atoms,
(b) a surfactant, and
(c) water,
wherein a mass ratio of a content of component (b) to a content of component (a), ((b)/(a)), in the stock solution (X) is 0.08 or more and 10 or less, and a content of ethanol is 8 mass% or less.

Sufficient improvement of the foaming performances including foam retentivity and foam feel of a squirted foam could not be achieved in either of the above-described Patent Literatures 1 and 2, and there remains room to be improved.

That is, the present invention relates to an oral cavity aerosol agent which can exhibit high foaming performances and well protect the gums.

The present inventors studied assiduously and found that the gums can well be protected when the squirted foam is thickened and the foam retentivity and the feeling that the gums are coated after rinsing are thereby improved by adding a nonionic surfactant and water to an oral cavity aerosol agent comprising a stock solution and a propellant comprising carbon dioxide as well as a specific amount of a specific monovalent alcohol and using a stock solution comprising a limited amount of ethanol, and thus an oral cavity aerosol agent with excellent usability can be obtained.

When the oral cavity aerosol agent of the present invention is used, high foam retentivity can be exhibited by squirting a thick foam. Further, after the foam is applied to the oral cavity and the oral cavity is rinsed, the foam remains well attached to the gums while being extensively spread in the oral cavity, resulting in a feeling that the gums are protected with a smooth film and a feeling that the gums are coated well.

### Brief Description of Drawings

Figure 1 is a photograph taken with a digital camera of a foam obtained by squirting the agent of Example 1.
Figure 2 is a photograph taken with a digital camera of a foam obtained by squirting the agent of Comparative Example 1.

### Detailed Description of the Invention

The present invention will be described in detail below.

The oral cavity aerosol agent of the present invention is an agent which is produced by filling a stock solution (X) and a propellant (Y) into an aerosol container and is applied into the oral cavity by squirting the agent when used. When carbon dioxide is added to the propellant (Y), part of the carbon dioxide is dissolved in the stock solution (X) and is also present inside the foam formed by the stock solution (X) after squirted from the aerosol container. Therefore, until after applying the agent to the oral cavity and rinsing the oral cavity, carbon dioxide can be efficiently delivered into the oral cavity while exhibiting excellent foaming performances.

Of note, performances of the foam formed by squirting the oral cavity aerosol agent of the present invention, which are achieved throughout a period from after squirting until after rinsing, such as "a sticky fine foam feel," "well attached to the gums while being spread extensively in the oral cavity," and "a feeling that the gum is protected with a smooth film is obtained," are also referred to as "excellent foaming performances" collectively.

The stock solution (X) included in the oral cavity aerosol agent of the present invention comprises 0.6 mass% or more and 10 mass% or less of a monovalent alcohol having 12 or more and 22 or less carbon atoms as a component (a). Adding the component (a) together with a component (b) in the presence of a component (c) described later enables formation of an α-gel having a lamellar structure and formation of a foam that exhibits excellent foaming performances throughout a period from after squirting until after rinsing.

The component (a) is preferably one or more selected from the group consisting of cetanol, stearyl alcohol, lauryl alcohol, myristyl alcohol, and behenyl alcohol, more preferably one or selected from the group consisting of cetanol and stearyl alcohol. It is further more preferable to contain both cetanol and stearyl alcohol as the component (a).

From a viewpoint of ensuring achievement of excellent foaming performances and a viewpoint of improving the feeling that gums are coated, it is preferable that the component (a) comprises at least stearyl alcohol. The content of stearyl alcohol in the stock solution (X) is preferably 0.6 mass% or more, more preferably 0.65 mass% or more, further more preferably 0.7 mass% or more; and is preferably 10 mass% or less, more preferably 7 mass% or less, further more preferably 4 mass% or less, even more preferably 1.5 mass% or less.

From a viewpoint of ensuring achievement of excellent foaming performances, the content of the component (a) in the stock solution (X) is 0.6 mass% or more, preferably 0.8 mass% or more, more preferably 1.5 mass% or more, further more preferably 1.8 mass% or more; and is 10 mass% or less, preferably 8 mass% or less, more preferably 5 mass% or less, further more preferably 3 mass% or less. Additionally, the content of the component (a) in the stock solution (X) is 0.6 mass% or more and 10 mass% or less, preferably from 0.8 to 8 mass%, more preferably from 1.5 to 5 mass%, further more preferably from 1.8 to 3 mass%.

The stock solution (X) included in the oral cavity aerosol agent of the present invention comprises a surfactant as a component (b). Adding the component (b) together with the above-described component (a) can ensure achievement of excellent foaming performances throughout a period from after squirting until after rinsing.

The surfactant of the component (b) used in the present invention is preferably one or more selected from the group consisting of nonionic surfactants, anionic surfactants, and ampholytic surfactants.

The nonionic surfactant is preferably an ester-type nonionic surfactant. Specific examples thereof include one or more selected from the group consisting of sorbitan fatty acid esters, polyoxyethylene sorbitan fatty acid esters, polyoxyethylene fatty acid esters, higher fatty acid glycerin esters, polyoxyethylene glyceryl monofatty acid esters, and sucrose fatty acid esters. Among these, from a viewpoint of imparting excellent emulsification stability to the stock solution (X), one or more selected from the group consisting of sorbitan fatty acid esters and polyoxyethylene sorbitan fatty acid esters are preferred, and combination use of sorbitan fatty acid esters and polyoxyethylene sorbitan fatty acid esters is more preferred.

The sorbitan fatty acid esters are derived preferably from fatty acids having 10 or more carbon atoms, more preferably from fatty acids having 12 or more carbon atoms; and is preferably from fatty acids having 20 or less carbon atoms, more preferably from fatty acids having 18 or less carbon atoms.

Specific examples thereof include one or more selected from the group consisting of sorbitan monocaprylate, sorbitan monoundecylate, sorbitan monolaurate, sorbitan monotridecylate, sorbitan monomyristate, sorbitan monopalmitate, sorbitan monooleate, sorbitan trioleate, sorbitan tetraoleate, sorbitan sesquioleate, sorbitan monostearate, sorbitan tristearate, and the like. Among these, one or more selected from the group consisting of sorbitan monooleate, sorbitan sesquioleate, and sorbitan monostearate are preferred, and sorbitan monostearate is more preferred.

The polyoxyethylene sorbitan fatty acid esters are derived preferably from fatty acids having 6 or more carbon atoms, more preferably from fatty acids having 12 or more carbon atoms; and is preferably from fatty acids having 22 or less carbon atoms, more preferably from fatty acids having 20 or less carbon atoms. The average number of moles of ethoxy groups to be added in a polyoxyethylene sorbitan fatty acid ester is preferably from 5 to 40 mol, more preferably from 10 to 25 mol, further more preferably from 15 to 25 mol.

Specific examples thereof include one or more selected from the group consisting of polyoxyethylene sorbitan monolaurate, polyoxyethylene sorbitan monomyristate, polyoxyethylene sorbitan monopalmitate, polyoxyethylene sorbitan monostearate, polyoxyethylene sorbitan monooleate, and the like. Among these, one or more polyoxyethylene sorbitan fatty acid esters selected from the group consisting of polyoxyethylene sorbitan monopalmitate, polyoxyethylene sorbitan monostearate, and polyoxyethylene sorbitan monooleate are preferred, and polyoxyethylene sorbitan monostearate is further more preferred.

An anionic surfactant preferably has a saturated or unsaturated hydrocarbon group having 8 to 18 carbon atoms as a lipophilic group, the hydrocarbon group having a straight or branched chain. The hydrocarbon group more preferably has 12 to 16 carbon atoms. As a hydrophilic group, carboxylic acid, sulfonic acid, phosphoric acid, or a salt thereof is preferable.

Specific examples thereof include one or more selected from the group consisting of alkyl sulfuric acid ester salts, polyoxyethylenealkyl ether sulfuric acid ester salts, alkyl sulfosuccinic acid salts, polyoxyethylenealkyl ether sulfosuccinic acid salts, alkyl ether carboxylic acid salts, alkyl phosphoric acid salts, polyoxyethylenealkyl ether phosphoric acid salts, fatty acid monoglyceride sulfuric acid salts, alkyl sulfoacetic acid salts, olefin sulfonic acid salts, and the like. Salts are preferably alkali metal salts or alkaline earth metal salts, and examples thereof include sodium salts, potassium salts, and magnesium salts. Among these, sodium salts are preferred.

Specific examples of ampholytic surfactants include one or more selected from the group consisting of betaine acetates such as lauryldimethylminobetaine acetate; imidazolinium betaine such as 2-alkyl-N-carboxymethyl-N-hydroxyethyl-N-imidazolium betaine; alkyl sulfobetaines such as lauryl sulfobetaine and lauryl hydroxysulfobetaine; cocamidoalkyl betaines such as cocamidopropyl betaine; long-chain alkyl imidazoline betaine salts such as N-alkyl-1-hydroxyethyl imidazoline betaine sodium.

From a viewpoint of ensuring achievement of excellent foaming performances throughout a period from after squirting until after rinsing, nonionic surfactants are preferred as the component (b).

From a viewpoint of ensuring achievement of excellent foaming performances, the content of the component (b) in the stock solution (X) is preferably 0.03 mass% or more, more preferably 0.08 mass% or more, further more preferably 0.15 mass% or more, even more preferably 0.3 mass% or more, even further more preferably 0.5 mass% or more; and is preferably 15 mass% or less, more preferably 5 mass% or less, further more preferably 3 mass% or less, even more preferably 1.8 mass% or less. Additionally, the content of the component (b) in the stock solution (X) is preferably 0.03 mass% or more and 15 mass% or less, more preferably from 0.08 to 5 mass%, further more preferably from 0.15 to 5 mass%, even more preferably from 0.3 to 3 mass%, even further more preferably from 0.5 to 1.8 mass%.

When the component (b) is a nonionic surfactant, the content thereof in the stock solution (X) is preferably 0.03 mass% or more, more preferably 0.08 mass% or more, further more preferably 0.15 mass% or more, even more preferably 0.3 mass% or more, even further more preferably 0.5 mass% or more; and is preferably 15 mass% or less, more preferably 5 mass% or less, further more preferably 3 mass% or less, even more preferably 1.8 mass% or less.

When the component (b) is an anionic surfactant, the content thereof in the stock solution (X) is preferably 0.4 mass% or less, more preferably 0.1 mass% or less, further more preferably 0.01 mass% or less. Of note, it is preferable that the stock solution (X) does not comprise an anionic surfactant as the component (b).

When the component (b) is an ampholytic surfactant, the content thereof in the stock solution (X) is preferably 0.03 mass% or more, more preferably 0.15 mass% or more, further more preferably 0.3 mass% or more; and is preferably 15 mass% or less, more preferably 3 mass% or less, further more preferably 1 mass% or less.

From a viewpoint of ensuring stability of the stock solution (X), a mass ratio of a content of the component (b) to a content of the component (a), ((b)/(a)), in the stock solution (X) is 0.08 or more, preferably 0.1 or more, more preferably 0.14 or more, further more preferably 0.23 or more, even more preferably 0.3 or more; and is 10 or less, preferably 7 or less, more preferably 3 or less, further more preferably 2 or less, even more preferably 1.5 or less, even further more preferably 0.8 or less. Additionally, the mass ratio of the content of the component (b) to the content of the component (a), ((b)/(a)), in the stock solution (X) is 0.08 or more and 10 or less, preferably from 0.1 to 7, more preferably from 0.14 to 3, further more preferably from 0.14 to 2, even more preferably from 0.23 to 1.5, even further more preferably from 0.3 to 0.8.

The stock solution (X) included in the oral cavity aerosol agent of the present invention comprises water as the component (c). The water as the component (c) in the present invention refers to not only purified water or the like added to the stock solution (X), but also all moistures contained in the stock solution (X), including a moisture contained in each component. When the stock solution (X) comprises water as the component (c), each component can be well dispersed or dissolved, and achievement of excellent foaming performances is ensured while adequate viscosity and good shape retainability are ensured as the stock solution (X).

A content of the component (c) in the stock solution (X) is preferably 50 mass% or more, more preferably 60 mass% or more, further more preferably 71 mass% or more, even more preferably 74 mass% or more; and is preferably 98 mass% or less, more preferably 95 mass% or less, further more preferably 85 mass% or less, even more preferably 80 mass% or less. Additionally, the content of the component (c) in the stock solution (X) is preferably 50 mass% or more and 98 mass% or less, more preferably from 60 to 95 mass%, further more preferably from 71 to 85 mass%, even more preferably from 74 to 80 mass%.

In the stock solution (X) included in the oral cavity aerosol agent of the present invention, the content of ethanol is 8 mass% or less. Thus, by limiting the content of ethanol in the stock solution (X), occurrence of defoaming and breaking of a foam formed after squirting can be effectively prevented and occurrence of unnecessary irritation can be suppressed.

The content of ethanol in the stock solution (X) is 8 mass% or less, preferably 6 mass% or less, more preferably 4 mass% or less, further more preferably 2 mass% or less, even more preferably 0.4 mass% or less. Alternatively, it is preferable that the stock solution (X) does not comprise ethanol.

When the stock solution (X) included in the oral cavity aerosol agent of the present invention comprises a binder, it is preferable that the content thereof is limited adequately. Thus, by avoiding an excessive content of a binder in the stock solution (X), the storage stability of the agent can be well ensured while maintaining adequate viscosity, and occurrence of unnecessary clogging in a container which interrupts squirting can be prevented effectively.

Specific examples of the binder include carboxymethyl cellulose sodium, hydroxyethyl cellulose, xanthan gum, hydroxypropyl cellulose, hydroxypropyl methyl cellulose, pectin, tragacanth gum, gum arabic, guar gum, karaya gum, locust bean gum, gellan gum, tamarind gum, psyllium seed gum, polyvinyl alcohol, chondroitin sulfate sodium, and methoxyethylene-maleic anhydride copolymers.

From a viewpoint of maintaining adequate viscosity and preventing occurrence of unnecessary clogging, the content of the binder in the stock solution (X) is preferably 1 mass% or less, more preferably 0.8 mass% or less, further more preferably 0.6 mass% or less, even more preferably 0.05 mass% or less. Alternatively, the stock solution (X) does not have to comprise a binder.

The stock solution (X) included in the oral cavity aerosol agent of the present invention may comprise one or more polyols (d) selected from the group consisting of glycerin, propylene glycol, ethylene glycol, sorbitol, xylitol, erythritol, reduced palatinose, and mannitol. Among these, the component (d) is preferably one or more selected from the group consisting of glycerin, sorbitol, xylitol, erythritol, reduced palatinose, and mannitol, more preferably one or more polyols selected from the group consisting of glycerin, sorbitol, xylitol, and erythritol.

The content of the component (d) in the stock solution (X) is preferably 30 mass% or less, more preferably 25 mass% or less, further more preferably 18 mass% or less, even more preferably 8 mass% or less. Alternatively, the stock solution (X) does not have to comprise the component (d).

The stock solution (X) included in the oral cavity aerosol agent of the present invention may comprise a pH modifier, a preservative, a medicinal ingredient, a flavor, a dye, and the like in addition to the above-described components, as long as the advantageous effects of the present invention are not inhibited.

From a viewpoint of exhibiting excellent foaming performances regardless of temperature changes and from a viewpoint of good squirting of the stock solution (X) from the aerosol container, the viscosity of the stock solution (X) at 25°C is preferably 500 mPa·s or higher, more preferably 700 mPa·s or higher, further more preferably 1 000 mPa·s or higher, even more preferably 1 300 mPa·s or higher, even further more preferably 1 800 mPa·s or higher; and is preferably 8 000 mPa·s or less, more preferably 6 000 mPa·s or less, further more preferably 5 000 mPa·s or less, even more preferably 4 000 mPa·s. Here, the viscosity of the stock solution (X) can be measured using a BM viscometer (manufactured by Toki Sangyo Co., Ltd.).

The propellant (Y) included in the oral cavity aerosol agent of the present invention preferably comprises carbon dioxide. Carbon dioxide can contribute to achievement of high foam retentivity while facilitating squirting of a thick foam.

From a viewpoint of ensuring achievement of excellent foaming performances, the content of carbon dioxide in the propellant (Y) is preferably 51 mass% or more, more preferably 80 mass% or more, further more preferably 90 mass% or more, even more preferably 95 mass% or more; and is preferably 100 mass% or less, more preferably 100 mass%.

As for the content of carbon dioxide, part thereof can be dissolved in the stock solution (X) during storage, but the content of carbon dioxide refers to the content in the propellant (Y) in a state before part of carbon dioxide is dissolved in the stock solution (X), specifically, before mixing the stock solution (X) and the propellant (Y) or before filling them into the aerosol container.

Of note, one or more gases selected from the group consisting of nitrogen; liquefied petroleum gases such as isobutane, normal butane, and a mixture thereof; liquefied gases such as dimethyl ether and isopentane may added to the propellant (Y) in addition to the above-described carbon dioxide, and these gases can be used as a gas mixed with carbon dioxide.

From a viewpoint of ensuring achievement of excellent foaming performances, a mass ratio of the stock solution (X) and the propellant (Y), ((X):(Y)), in the oral cavity aerosol agent of the present invention is preferably from 95:5 to 99:1, more preferably from 97:3 to 98.5:1.5.

From a viewpoint of ensuring achievement of excellent foaming performances, a mass ratio of the content of carbon dioxide in the propellant (Y) to the content of the component (a) in the stock solution (X), [(carbon dioxide)/(a)], in the oral cavity aerosol agent of the present invention is preferably 0.02 or more, more preferably 0.18 or more, further more preferably 0.2 or more, even more preferably 0.25 or more, even further more preferably 0.35 or more; and is preferably 5 or less, more preferably 3 or less, further more preferably 2.5 or less, even more preferably 1.5 or less.

From a viewpoint of ensuring achievement of excellent foaming performances, a mass ratio of the content of carbon dioxide in the propellant (Y) to the content of the component (b) in the stock solution (X), [(carbon dioxide)/(b)], in the oral cavity aerosol agent of the present invention is preferably 0.02 or more, more preferably 0.25 or more, further more preferably 0.5 or more, even more preferably 0.8 or more; and is preferably 25 or less, more preferably 20 or less, further more preferably 12 or less, even more preferably 6 or less.

The oral cavity aerosol agent of the present invention is obtained by compressing the propellant (Y) and filling it into an aerosol container. From a viewpoint that a foam having excellent performances can be formed after squirted from the container when used, the aerosol container is preferably equipped with a foam discharge port. When the oral cavity aerosol agent of the present invention is manufactured, the above-described stock solution (X) may be prepared, then filled into the aerosol container together with the propellant (Y), and encapsulated by pressurizing as necessary.

Of note, from a viewpoint of ensuring achievement of excellent foaming performances, the pressure in the aerosol container at 25°C is preferably from 0.5 to 0.9 MPa, more preferably from 0.7 to 0.8 MPa.

The oral cavity aerosol agent of the present invention is preferably applied to an intended site after once placing the content squirted from the container when used on a toothbrush, a fingertip, or the like. The appropriate dose of the oral cavity aerosol agent of the present invention per use is preferably from 0.4 to 1.5 g, more preferably from 0.5 to 1.0 g.

The present invention further discloses the following oral cavity aerosol agents based on the above-described embodiments:
[1] An oral cavity aerosol agent comprising a stock solution (X) and a propellant (Y), the stock solution (X) comprising the following components (a), (b), and (c):
   (a) 0.6 mass% or more and 10 mass% or less of a monovalent alcohol having 12 or more and 22 and less carbon atoms,
   (b) a surfactant, and
   (c) water,
   wherein a mass ratio of the content of the component (b) to the content of the component (a), ((b)/(a)), in the stock solution (X) is 0.08 or more and 10 or less, and the content of ethanol is 8 mass% or less.
[2] The oral cavity aerosol agent according to the above [1], wherein the component (a) is preferably cetanol and one or more alcohols selected from the group consisting of stearyl alcohol, lauryl alcohol, myristyl alcohol, and behenyl alcohol, more preferably one or more alcohols selected from the group consisting of cetanol and stearyl alcohol, and it is further more preferable that the oral cavity aerosol agent comprises both cetanol and stearyl alcohol as the component (a).
[3] The oral cavity aerosol agent according to the above [1] or [2], wherein the content of the component (a) in the stock solution (X) is preferably 0.8 mass% or more, more preferably 1.5 mass% or more, further more preferably 1.8 mass% or more; and is 10 mass% or less, preferably 8 mass% or less, more preferably 5 mass% or less, further more preferably 3 mass% or less.
[4] The oral cavity aerosol agent according to any one of the above [1] to [3], wherein the component (b) is preferably one or more selected from the group consisting of nonionic surfactants, anionic surfactants, and ampholytic surfactants, more preferably a nonionic surfactant, further more preferably an ester-type nonionic surfactant, even more preferably one or more selected from the group consisting of sorbitan fatty acid esters and polyoxyethylene sorbitan fatty acid esters, and it is even more preferable that the oral cavity aerosol agent comprises both a sorbitan fatty acid ester and a polyoxyethylene sorbitan fatty acid ester.
[5] The oral cavity aerosol agent according to any one of the above [1] to [4], wherein the content of the component (b) in the stock solution (X) is preferably 0.03 mass% or more, more preferably 0.08 mass% or more, further more preferably 0.15 mass% or more, even more preferably 0.3 mass% or more, even further more preferably 0.5 mass% or more; and is preferably 15 mass% or less, more preferably 5 mass% or less, further more preferably 3 mass% or less, even more preferably 1.8 mass% or less.
[6] The oral cavity aerosol agent according to any one of the above [1] to [5], wherein the mass ratio of the content of the component (b) to the content of the component (a), ((b)/(a)), in the stock solution (X) is preferably 0.1 or more, more preferably 0.14 or more, further more preferably 0.23 or more, even more preferably 0.3 or more; and is preferably 7 or less, more preferably 3 or less, further more preferably 2 or less, even more preferably 1.5 or less, even further more preferably 0.8 or less.
[7] The oral cavity aerosol agent according to any one of the above [1] to [6], wherein the content of the component (c) in the stock solution (X) is preferably 50 mass% or more, more preferably 60 mass% or more, further more preferably 71 mass% or more, even more preferably 74 mass% or more; and is preferably 98 mass% or less, more preferably 95 mass% or less, further more preferably 85 mass% or less, even more preferably 80 mass% or less.
[8] The oral cavity aerosol agent according to any one of the above [1] to [7], wherein the content of ethanol in the stock solution (X) is preferably 6 mass% or less, more preferably 4 mass% or less, further more preferably 2 mass% or less, even more preferably 0.4 mass% or less, or it is preferable that the stock solution (X) does not comprise ethanol.
[9] The oral cavity aerosol agent according to any one of the above [1] to [8], wherein the content of the binder in the stock solution (X) is preferably 1 mass% or less, more preferably 0.8 mass% or less, further more preferably 0.6 mass% or less, even more preferably 0.05 mass% or less, or the stock solution (X) does not have to comprise a binder.
[10] The oral cavity aerosol agent according to any one of the above [1] to [9], wherein the stock solution (X) may comprise one or more polyols (d) selected from the group consisting of glycerin, propylene glycol, ethylene glycol, sorbitol, xylitol, erythritol, reduced palatinose, and mannitol, and the component (d) is preferably one or more polyols selected from the group consisting of glycerin, sorbitol, xylitol, erythritol, reduced palatinose, and mannitol, more preferably one or more polyols selected from the group consisting of glycerin, sorbitol, xylitol, and erythritol.
[11] The oral cavity aerosol agent according to the above [10], wherein the content of the component (d) in the stock solution (X) is preferably 30 mass% or less, more preferably 25 mass% or less, further more preferably 18 mass% or less, even more preferably 8 mass% or less, or the stock solution (X) does not have to comprise the component (d).
[12] The oral cavity aerosol agent according to any one of the above [1] to [11], wherein the propellant (Y) preferably comprises carbon dioxide, and the content of the carbon dioxide in the propellant (Y) is preferably 51 mass% or more, more preferably 80 mass% or more, further more preferably 90 mass% or more, even more preferably 95 mass% or more; and is preferably 100 mass% or less, more preferably 100 mass%.
[13] The oral cavity aerosol agent according to any one of the above [1] to [12], wherein a mass ratio of stock solution (X) and the propellant (Y), (X):(Y), is preferably from 95:5 to 99:1, more preferably from 97:3 to 98.5:1.5.
[14] The oral cavity aerosol agent according to the above [12] or [13], wherein a mass ratio of the content of carbon dioxide in the propellant (Y) to the content of component (a), [(carbon dioxide)/(a)], in the stock solution (X) is preferably 0.02 or more, more preferably 0.18 or more, further more preferably 0.2 or more, even more preferably 0.25 or more, even further more preferably 0.35 or more; and is preferably 5 or less, more preferably 3 or less, further more preferably 2.5 or less, even more preferably 1.5.
[15] The oral cavity aerosol agent according to any one of the above [12] to [14], wherein a mass ratio of the content of carbon dioxide in the propellant (Y) to the content of component (b) in the stock solution (X), [(carbon dioxide)/(b)], preferably 0.02 or more, more preferably 0.25 or more, further more preferably 0.5 or more, even more preferably 0.8 or more; and is preferably 25 or less, more preferably 20 or less, further more preferably 12 or less, even more preferably 6 or less.

### Examples

The present invention will specifically be described by way of examples. Of note, the content of each component in the tables is expressed in mass% unless otherwise specified.

### [Examples 1 to 33, Comparative Examples 1 to 7]

To manufacture each agent, each stock solution (X) was prepared in accordance with a prescription in Tables 1 to 6 and filled into an aerosol container (manufactured by Takeuchi Press Industries Co., Ltd.), a propellant (Y) was encapsulated at a ratio shown in Tables 1 to 6, and a pressure in the container at 25°C was 0.9 MPa.

The obtained agents were subjected to measurement and assessment respectively in accordance with the following methods.

The results are shown in Tables 1 to 6.

### <Viscosity of stock solution (X) at 25°C>

Viscosity was measured using a BM viscometer (rotor No. 3, 12 rpm, 1 minute) (manufactured by Toki Sangyo Co., Ltd.).

### <Foam retentivity>

An O ring with an inside diameter of 21.7 mm was placed on a sieve (No. 5.5, 3350 µm), and then 1 g of each agent was squirted from the container and allowed to stand in the O ring. After the agent was allowed to stand, whether the squirted foam agent was passed through the sieve and dripped downwards was observed from the above of the sieve, and the time (seconds) from immediately after discharge until dripping of the foam agent was measured and used as a measure of an assessment.

### <Assessment of feeling that gums are coated>

Feeling that the gums are protected with a smooth film (feeling that the gums are coated) was assessed. Specifically, each agent was squirted from the container onto a toothbrush to form approximately 0.5 g of a foam, brushing for 3 minutes and rinsing were performed, and the feel surrounding the gums that was felt with the tongue was assessed in accordance with the following criteria.

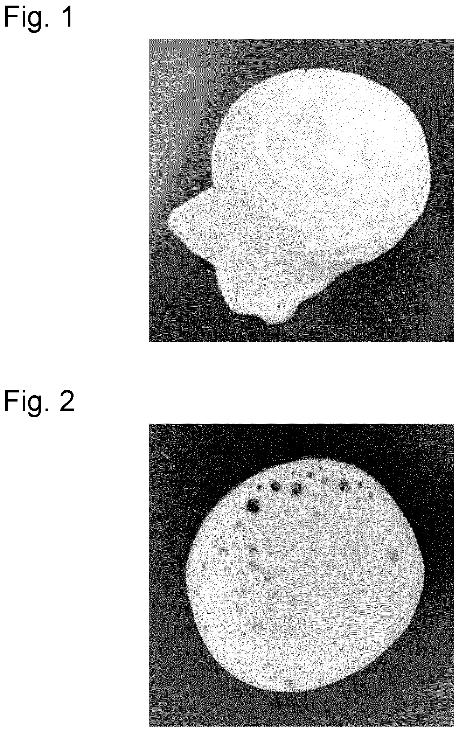

Figure 1 is a photograph of the squirted agent of Example 1, and Figure 2 is a photograph of the squirted agent of Comparative Example 1.
3: Feeling that the gums are protected with a smooth film was sufficiently obtained.
2: Feeling that the gums are protected with a smooth film was slightly sufficiently obtained.
1: Feeling that the gums are protected with a smooth film was rarely obtained.

Of note, after the agents obtained in Examples 1 and 16 were further stored at room temperature (25°C) for 1 year, foam retentivity and feeling that the gums are coated were measured and assessed again in the same manner as above, and the following results were obtained.
- Example 1:
   Foam retentivity, >300 seconds; feeling that gums are coated, 3
- Example 16:
   Foam retentivity: >300 seconds; feeling that gums are coated, 2

**[Table 1]**

| | | | Example 1 | Example 2 | Example 3 | Example 4 | Example 5 | Example 6 | Example 7 | Example 8 | Example 9 |
|---|---|---|---|---|---|---|---|---|---|---|---|
| Stock solution (X) | Content (mass%) in stock solution (X) (100 mass%) | | | | | | | | | | |
| | (a) | Cetanol | 1.2 | 5.4 | 0.45 | 0.6 | 3.6 | 1.2 | 1.2 | 1.2 | 1.2 |
| | | Stearyl alcohol | 0.8 | 3.6 | 0.3 | 0.4 | 2.4 | 0.8 | 0.8 | 0.8 | 0.8 |
| | (b) | Sorbitan monostearate | 1.08 | 1.08 | 1.08 | 1.08 | 1.08 | 1.08 | 0 | 0 | 1.08 |
| | | Polyoxyethylene sorbitan monostearate | 0.18 | 0.18 | 0.18 | 0.18 | 0.18 | 0.18 | 0.18 | 0.18 | 0 |
| | | Sorbitan monooleate | 0 | 0 | 0 | 0 | 0 | 0 | 1.08 | 0 | 0 |
| | | Sorbitan sesquioleate | 0 | 0 | 0 | 0 | 0 | 0 | 0 | 1.08 | 0 |
| | | Polyoxyethylene sorbitan monooleate | 0 | 0 | 0 | 0 | 0 | 0 | 0 | 0 | 0.18 |
| | (d) | Concentrated glycerin | 10 | 10 | 10 | 10 | 10 | 10 | 10 | 10 | 10 |
| | | Sorbitol | 10 | 10 | 10 | 10 | 10 | 10 | 10 | 10 | 10 |
| | | Propylene glycol | 0 | 0 | 0 | 0 | 0 | 0 | 0 | 0 | 0 |
| | (c) | Purified water | 76.74 | 69.74 | 77.99 | 77.74 | 72.74 | 75.74 | 76.74 | 76.74 | 76.74 |
| | | Ethanol | 0 | 0 | 0 | 0 | 0 | 1 | 0 | 0 | 0 |
| Propellant (Y) | Content (mass%) in propellant (Y) (100 mass%) | | | | | | | | | | |
| | Carbon dioxide | | 100 | 100 | 100 | 100 | 100 | 100 | 100 | 100 | 100 |
| Aerosol | (Stock solution (X)):(Propellant (Y)) | | 98:2 | 98:2 | 98:2 | 98:2 | 98:2 | 98:2 | 98:2 | 98:2 | 98:2 |
| ((b)/(a)) | | | 0.63 | 0.14 | 1.68 | 1.26 | 0.21 | 0.63 | 0.63 | 0.63 | 0.63 |
| [(Carbon dioxide)/(a)] | | | 0.98 | 0.22 | 2.61 | 1.96 | 0.33 | 0.98 | 0.98 | 0.98 | 0.98 |
| [(Carbon dioxide)/(b)] | | | 1.56 | 1.56 | 1.56 | 1.56 | 1.56 | 1.56 | 1.56 | 1.56 | 1.56 |
| Stock solution viscosity (mPa·s) | | | 2620 | 6540 | 1740 | 1810 | 4040 | 2430 | 1800 | 1730 | 930 |
| Foam retentivity (seconds) | | | 175 | >300 | 170 | 170 | >300 | 180 | >300 | >300 | >300 |
| Feeling that gums are coated | | | 3 | 3 | 2 | 3 | 3 | 3 | 3 | 3 | 3 |

**[Table 2]**

| | | | Example 10 | Example 11 | Example 12 | Example 13 | Example 14 | Example 15 | Example 16 | Example 17 | Example 18 | Example 19 | Example 20 |
|---|---|---|---|---|---|---|---|---|---|---|---|---|---|
| Stock solution (X) | Content (mass%) in stock solution (X) (100 mass%) | | | | | | | | | | | | |
| | (a) | Cetanol | 0.6 | 0.6 | 0.6 | 1.2 | 1.2 | 2 | 0 | 0.8 | 0 | 1.2 | 1.2 |
| | | Stearyl alcohol | 0.4 | 0.4 | 0.4 | 0.8 | 0.8 | 0 | 2 | 0 | 1.2 | 0.8 | 0.8 |
| | | Myristyl alcohol | 0 | 0 | 0 | 0 | 0 | 0 | 0 | 1.2 | 0 | 0 | 0 |
| | | Behenyl alcohol | 0 | 0 | 0 | 0 | 0 | 0 | 0 | 0 | 0.8 | 0 | 0 |
| | (b) | Sorbitan monostearate | 0.110 | 0.220 | 2.16 | 1.08 | 1.08 | 1.08 | 1.08 | 1.08 | 1.08 | 1.08 | 1.08 |
| | | Polyoxyethylene sorbitan monostearate | 0.018 | 0.036 | 0.36 | 0.18 | 0.18 | 0.18 | 0.18 | 0.18 | 0.18 | 0.18 | 0.18 |
| | (d) | Concentrated glycerin | 10 | 10 | 10 | 0 | 0 | 10 | 10 | 10 | 10 | 10 | 10 |
| | | Sorbitol | 10 | 10 | 10 | 0 | 0 | 10 | 10 | 10 | 10 | 10 | 10 |
| | | Propylene glycol | 0 | 0 | 0 | 0 | 10 | 0 | 0 | 0 | 0 | 0 | 0 |
| | (c) | Purified water | 78.872 | 78.744 | 76.48 | 96.74 | 86.74 | 76.74 | 76.74 | 76.74 | 76.74 | 76.24 | 76.24 |
| | | Xanthan gum | 0 | 0 | 0 | 0 | 0 | 0 | 0 | 0 | 0 | 0.5 | 0 |
| | | Hydroxypropyl methyl cellulose | 0 | 0 | 0 | 0 | 0 | 0 | 0 | 0 | 0 | 0 | 0.5 |
| | | Ethanol | 0 | 0 | 0 | 0 | 0 | 0 | 0 | 0 | 0 | 0 | 0 |
| Propellant (Y) | Content (mass%) in propellant (Y) (100 mass%) | | | | | | | | | | | | |
| | Carbon dioxide | | 100 | 100 | 100 | 100 | 100 | 100 | 100 | 100 | 100 | 100 | 100 |
| Aerosol | (Stock solution (X)):(Propellant (Y)) | | 98:2 | 98:2 | 98:2 | 98:2 | 98:2 | 98:2 | 98:2 | 98:2 | 98:2 | 98:2 | 98:2 |
| ((b)/(a)) | | | 0.13 | 0.26 | 2.52 | 0.63 | 0.63 | 0.63 | 0.63 | 0.63 | 0.63 | 0.63 | 0.63 |
| [(Carbon dioxide)/(a)] | | | 1.96 | 1.96 | 1.96 | 0.98 | 0.98 | 0.98 | 0.98 | 0.98 | 0.98 | 0.98 | 0.98 |
| [(Carbon dioxide)/(b)] | | | 15.31 | 7.66 | 0.78 | 1.56 | 1.56 | 1.56 | 1.56 | 1.56 | 1.56 | 1.56 | 1.56 |
| Stock solution viscosity (mPa·s) | | | 980 | 920 | 2670 | 2000 | 3000 | 2480 | 3880 | 1210 | 3000 | 2500 | 4710 |
| Foam retentivity (seconds) | | | 90 | 100 | 240 | 200 | 175 | 220 | 235 | 200 | 165 | >300 | >300 |
| Feeling that gums are coated | | | 2 | 2 | 3 | 3 | 3 | 3 | 3 | 2 | 3 | 3 | 3 |

**[Table 3]**

| | | | Comparative Example 1 | Comparative Example 2 | Comparative Example 3 | Comparative Example 4 | Comparative Example 5 |
|---|---|---|---|---|---|---|---|
| Stock solution (X) | Content (mass%) in stock solution (X) (100 mass%) | | | | | | |
| | (a) | Cetanol | 0 | 0 | 0.6 | 7.2 | 1.2 |
| | | Stearyl alcohol | 0 | 0 | 0.4 | 4.8 | 0.8 |
| | (b) | Sorbitan monostearate | 0 | 1.08 | 10.8 | 1.08 | 0 |
| | | Polyoxyethylene sorbitan monostearate | 0.18 | 0.18 | 1.8 | 0.18 | 0 |
| | (d) | Concentrated glycerin | 10 | 10 | 10 | 10 | 10 |
| | | Sorbitol | 10 | 10 | 10 | 10 | 10 |
| | (c) | Purified water | 79.82 | 78.74 | 66.40 | 66.74 | 78 |
| | | Ethanol | 0 | 0 | 0 | 0 | 0 |
| Propellant (Y) | Content (mass%) in propellant (Y) (100 mass%) | | | | | | |
| | Carbon dioxide | | 100 | 100 | 100 | 100 | 100 |
| Aerosol | (Stock solution (X)):(Propellant (Y)) | | 98:2 | 98:2 | 98:2 | 98:2 | 98:2 |
| ((b)/(a)) | | | - | - | 12.60 | 0.11 | 0 |
| [(Carbon dioxide)/(a)] | | | - | - | 1.96 | 0.16 | 0.98 |
| [(Carbon dioxide)/(b)] | | | 10.89 | 1.56 | 0.16 | 1.56 | - |
| Stock solution viscosity (mPa·s) | | | 110 | 190 | -^{*1} | 9340 | -^{*2} |
| Foam retentivity (seconds) | | | 3 | >300 | -^{*3} | -^{*3} | -^{*3} |
| Feeling that gums are coated | | | 1 | 1 | -^{*3} | -^{*3} | -^{*3} |

| | | | | | | | |
|---|---|---|---|---|---|---|---|
| *1: Could not be measured because the upper measuring limit was exceeded. *2: Could not be measured because of syneresis. *3: Not measurable | | | | | | | |

**[Table 4]**

| | | | Comparative Example 6 |
|---|---|---|---|
| Stock solution (X) | Content (mass%) in stock solution (X) (100 mass%) | | |
| | (a) | Cetanol | 1.2 |
| | | Stearyl alcohol | 0.8 |
| | (b) | Sorbitan monostearate | 1.08 |
| | | Polyoxyethylene sorbitan monostearate | 0.18 |
| | (d) | Concentrated glycerin | 10 |
| | | Sorbitol | 10 |
| | (c) | Purified water | 66.74 |
| | | Ethanol | 10 |
| Propellant (Y) | Content (mass%) in propellant (Y) (100 mass%) | | |
| | Carbon dioxide | | 100 |
| Aerosol | (Stock solution (X)):(Propellant (Y)) | | 98:2 |
| ((b)/(a)) | | | 0.63 |
| [(Carbon dioxide)]/(a)] | | | 0.98 |
| [(Carbon dioxide)/(b)] | | | 1.56 |
| Stock solution viscosity (mPa·s) | | | 2500 |

**[Table 5]**

| | | | Example 21 | Example 22 | Example 23 | Example 24 | Example 25 | Example 26 | Example 27 | Example 28 | Example 29 | Comparative Example 7 |
|---|---|---|---|---|---|---|---|---|---|---|---|---|
| Stock solution (X) | Content (mass%) in stock solution (X) (100 mass%) | | | | | | | | | | | |
| | (a) | Cetanol | 1.2 | 1.2 | 1.2 | 1.2 | 1.2 | 1.2 | 1.2 | 1.2 | 1.2 | 1.2 |
| | | Stearyl alcohol | 0.8 | 0.8 | 0.8 | 0.8 | 0.8 | 0.8 | 0.8 | 0.8 | 0.8 | 0.8 |
| | (b) | Sorbitan monostearate | 0 | 0 | 0 | 1.08 | 1.08 | 1.08 | 1.08 | 1.08 | 1.08 | 1.08 |
| | | Polyoxyethylene sorbitan monostearate | 0.18 | 0.18 | 0.18 | 0.18 | 0.18 | 0.18 | 0.18 | 0.18 | 0.18 | 0.18 |
| | | Polyoxyethylene hydrogenated caster oil | 1.08 | 0 | 0 | 0 | 0 | 0 | 0 | 0 | 0 | 0 |
| | | Polyglyceryl myristate | 0 | 1.08 | 0 | 0 | 0 | 0 | 0 | 0 | 0 | 0 |
| | | Sucrose palmitic acid ester | 0 | 0 | 1.08 | 0 | 0 | 0 | 0 | 0 | 0 | 0 |
| | (d) | Concentrated glycerin | 10 | 10 | 10 | 10 | 10 | 10 | 10 | 10 | 10 | 10 |
| | | Sorbitol | 10 | 10 | 10 | 10 | 10 | 10 | 10 | 10 | 10 | 10 |
| | (c) | Purified water | 76.74 | 76.74 | 76.74 | 76.74 | 76.74 | 76.74 | 76.74 | 76.74 | 76.74 | 76.74 |
| | | Ethanol | 0 | 0 | 0 | 0 | 0 | 0 | 0 | 0 | 0 | 0 |
| Propellant | Content (mass%) in propellant (Y) (100 mass%) | | | | | | | | | | | |
| | Carbon dioxide | | 100 | 100 | 100 | 100 | 100 | 100 | 75 | 50 | 25 | 0 |
| | Nitrogen | | 0 | 0 | 0 | 0 | 0 | 0 | 25 | 50 | 75 | 100 |
| Aerosol | (Stock solution (X)):(Propellant (Y)) | | 98:2 | 98:2 | 98:2 | 97.5:2.5 | 98.5:1.5 | 99:1 | 98:2 | 98:2 | 98:2 | 98:2 |
| ((b)/(a)) | | | 0.63 | 0.63 | 0.63 | 0.63 | 0.63 | 0.63 | 0.63 | 0.63 | 0.63 | 0.63 |
| [(Carbon dioxide)/(a)] | | | 0.98 | 0.98 | 0.98 | 1.22 | 0.74 | 0.50 | 0.74 | 0.49 | 0.25 | - |
| [(Carbon dioxide)/(b)] | | | 1.56 | 1.56 | 1.56 | 1.93 | 1.17 | 0.79 | 1.17 | 0.78 | 0.39 | - |
| Stock solution viscosity (mPa·s) | | | 3080 | 260 | 2770 | 2620 | 2620 | 2620 | 2620 | 2620 | 2620 | 2620 |
| | | | | | | | | | | | | |
| | | | | | | | | | | | | |
| Foam retentivity (seconds) | | | >300 | >300 | >300 | 200 | 180 | 170 | 180 | 160 | 90 | 25 |
| Feeling that gums are coated | | | 3 | 3 | 3 | 3 | 3 | 3 | 2 | 2 | 2 | 1 |

**[Table 6]**

| | | | Example 30 | Example 31 | Example 32 | Example 33 |
|---|---|---|---|---|---|---|
| Stock solution (X) | Content (mass%) in stock solution (X) (100 mass%) | | | | | |
| | (a) | Cetanol | 1.2 | 1.2 | 1.2 | 1.2 |
| | | Stearyl alcohol | 0.8 | 0.8 | 0.8 | 0.8 |
| | (b) | Sorbitan monostearate | 1.08 | 1.08 | 1.08 | 1.08 |
| | | Polyoxyethylene sorbitan monostearate | 0.18 | 0.18 | 0.18 | 0.18 |
| | | Cocamidopropyl betaine | 0 | 0 | 0 | 0.5 |
| | (d) | Concentrated glycerin | 30 | 0 | 0 | 0 |
| | | Xylitol | 0 | 10 | 0 | 0 |
| | | Erythritol | 0 | 0 | 10 | 10 |
| | (c) | Purified water | 66.740 | 86.74 | 86.74 | 86.24 |
| | | Ethanol | 0 | 0 | 0 | 0 |
| Propellant (Y) | Content (mass%) in propellant (Y) (100 mass%) | | | | | |
| | Carbon dioxide | | 100 | 100 | 100 | 100 |
| Aerosol | (Stock solution (X)):(Propellant (Y)) | | 98:2 | 98:2 | 98:2 | 98:2 |
| ((b)/(a)) | | | 0.63 | 0.63 | 0.63 | 0.63 |
| [(Carbon dioxide)/(a)] | | | 0.98 | 0.98 | 0.98 | 0.98 |
| [(Carbon dioxide)/(b)] | | | 1.56 | 1.56 | 1.56 | 1.56 |
| Stock solution viscosity (mPa·s) | | | 4020 | 2310 | 2160 | 3870 |
| Foam retentivity (seconds) | | | 240 | >300 | >300 | >300 |
| Feeling that gums are coated | | | 3 | 2 | 2 | 3 |

### <Assessment of irritating properties in oral cavity>

Each of the agents obtained in Examples 1 and 6 and Comparative Example 6 was squirted from each container to form approximately 0.5 g of a foam on the toothbrush, brushing for 3 minutes and rinsing were performed, and then the presence/absence of irritation in the oral cavity was assessed.

As a result, irritation was not felt in Example 1 and Example 6, but irritation was felt in Comparative Example 6.

## Claims

1. An oral cavity aerosol agent comprising a stock solution (X) and a propellant (Y), the stock solution (X) comprising the following components (a), (b), and (c):
(a) 0.6 mass% or more and 10 mass% or less of a monovalent alcohol having 12 or more and 22 and less carbon atoms,
(b) a surfactant, and
(c) water,
wherein a mass ratio of a content of the component (b) to a content of the component (a), ((b)/(a)), in the stock solution (X) is 0.08 or more and 10 or less, and the content of ethanol is 8 mass% or less.

2. The oral cavity aerosol agent according to claim 1, wherein the component (b) is one or more selected from the group consisting of sorbitan fatty acid esters and polyoxyethylene sorbitan fatty acid esters.

3. The oral cavity aerosol agent according to claim 1 or 2, wherein a content of a binder in the stock solution (X) is 1 mass% or less.

4. The oral cavity aerosol agent according to any one of claims 1 to 3, wherein the propellant (Y) comprises carbon dioxide.

5. The oral cavity aerosol agent according to claim 4, wherein a mass ratio of a content of carbon dioxide in the propellant (Y) to the content of the component (a) in the stock solution (X), [(carbon dioxide)/(a)], is 0.02 or more and 5 or less.

6. The oral cavity aerosol agent according to claim 4 or 5, wherein a mass ratio of the content of carbon dioxide in the propellant (Y) to the content of the component (b) in the stock solution (X), [(carbon dioxide)/(b)], is 0.02 or more and 25 or less.
